# EUROPEAN PATENT APPLICATION

(11) **EP 2 277 531 A1**
(43) Date of publication of application: **26.01.2011**
(21) Application number: 10179661.3
(22) Date of filing: 04.03.2003
(51) Int. Cl.: A61K 38/51, A61P 25/00

(54) **Treatment of cns damage**

(30) Priority: 04.03.2002 GB 0205022
(62) Divisional of application: 03706754.3
(71) Applicant: Cambridge Enterprise Limited, Trinity Lane Cambridge Cambridgeshire CB2 1TN (GB); KINGS COLLEGE LONDON, London WC2R 2LS (GB)
(72) Inventor: McMahon, Stephen, Brendan, London, Greater London SE1 1UL (GB); Bradbury, Elizabeth, Jane, London, Greater London SE1 1UL (GB); Fawcett, James, Cambridge, Cambridgeshire CB2 2PY (GB)
(74) Representative: Hayes, Emily Anne Luxford

(57) **Abstract**

The invention provides a method of promoting neuronal plasticity in the CNS of a mammal, the method comprising administering to the CNS of the mammal an agent that reduces the inhibitory properties of chondroitin sulphate proteoglycans. Preferred agents are chondroitinases and sulfatases, e.g. chondroitinase ABC. Also provided are methods of identifying further agents.

## Description

### Field of the Invention

The invention relates to materials and methods for the treatment of damage to the central nervous system (CNS), in particular to methods and materials to promote neuronal plasticity.

### Background

"Plasticity" is a term that refers to the ability of the nervous system to change the connections between neurones so as to alter the function of the brain or spinal cord, often in response to sensory or behavioural stimuli or to damage. The term encompasses the activation of synapses that were structurally present but inactive, the strengthening and weakening of synapses, and the making and breaking of synapses. New synapses are formed by sprouting of nerve terminals to send a new branch of the terminal to a new synaptic site. Synapses are withdrawn by the loss of contact of a terminal with its target neurone, usually followed by retraction of the terminal branch.

After stroke, head injury, surgery or other insults to the brain there is a period during which there is partial recovery of some of the neurological function that has been lost. This is thought to be largely due to rearrangement of connections in the cortex and subcortical areas of the brain. Thus the function that used to be performed by the part of the brain that has been lost may be partially taken over by part of the surviving brain. This rearrangement of connections and functions is an example of plasticity. A treatment that could enhance plasticity after brain injury, stroke, neurodegenerative disease, multiple sclerosis or any other disease that causes loss of neurones or neuronal connections would probably enhance recovery of function in patients, generally in association with physiotherapy and other forms of training.

Studies have been conducted, by the inventors and others, into axon regeneration following axotomy in the CNS. These studies, described in greater detail below, have focussed principally on the spinal cord, but have also been carried out on the nigrostriatial tract of the brain. Although relating to the promotion of axon regeneration and not neuronal plasticity, these studies provide useful background to the invention.

A structure called the glial scar develops wherever the brain or spinal cord is injured. This reactive scarring process involves the glial cell types astrocytes, microglia, oligodendrocyte precursors and meningeal cells. Axons that are cut as a result of injury in the CNS fail to regenerate, and the tips of the non-regenerating axon are found in the glial scar. Various experiments over many years have demonstrated that the glial scar is inhibitory to axon regeneration (Fawcett and Asher, 1999).

A series of in vitro experiments have been carried out to determine which molecules produced by glial cells in the glial scar block axon regeneration. It was shown that the main inhibitory molecules are chondroitin sulphate proteoglycans (CSPGs) (Smith-Thomas et al., 1994; Fawcett and Asher, 1999).

Chondroitin sulphate proteoglycans are molecules made up of a protein core to which one or more long sulphated sugar chains are attached. The sugar chains, known as glycosaminoglycan chains, are made of repeating discaccharides mainly consisting of N-acetylgalactosamine and glucuronic acid. They are sulphated in a variety of positions.

In tissue culture experiments it has been shown that chondroitinase (which digests away the sugar chains), sodium chlorate (which inhibits sulphation of the sugar chains) and beta-D-xylosides (which prevent the sugar chains attaching to the protein core) all made CNS glia less inhibitory (Smith-Thomas et al., 1995 and others).

It has also been shown that chondroitin sulphate proteoglycans are upregulated at sites of CNS injury (DeWitt et al., 1994). Many of the individual types of proteoglycan and the cell types that produce them have since been identified: NG2, versican, neurocan, brevican, phosphacan and aggrecan are inhibitory chondroitin sulphate proteoglycans found in CNS injuries (Fawcett and Asher, 1999; Fidler et al., 1999; Asher et al., 1999; Asher et al., 2000).

Based on tissue culture evidence that removing glycosaminoglycan chains with chondroitinase can make chondroitin sulphate proteoglycans less inhibitory, and on the fact that proteoglycans are present in high concentration in the glial scar around CNS injuries, chondroitinase was applied to a brain injury that cut the axons of the nigrostriatal tract. The chondroitinase treatment allowed a proportion of the cut axons to regenerate back to their target (Moon et al., 2001). Similarly, depletion of CSPG-producing oligodendrite precursors, using cytosine arabinoside (an anti-mitotic agent), also promoted axon regeneration (Rhodes et al., 2000).

Using chondroitinase, axon regeneration has also been shown in vivo in animals after experimentally induced spinal cord damage (Bradbury et al., 2000a, 2002).

Following the successful promotion of axon regeneration in the brain, an in vivo experiment (reported in the Examples herein) was conducted in which chondroitinase was administered to spinal cord injuries, using the same protocol as had been used for the nigrostriatal tract experiments. The treatment promoted the regeneration of both sensory and motor axons, and caused recovery of behavioural function in sensory-motor tests of forelimb function (Bradbury et al., 2002). These forelimb behavioural tests recover very slowly in untreated rats.

The inventors hypothesised, however, that the behavioural recovery in chondroitinase treated animals was so rapid and so complete that the recovery might be attributable, at least in part, to rearrangement of the connections of the unlesioned axons in the cord, i.e. neuronal plasticity.

In particular, the inventors hypothesised that the large behavioural recovery seen in beam walking and grid walking tasks following chondroitinase treatment of the spinal cord after C4 dorsal column lesions was due to enhanced plasticity as much as to axon regeneration. The bases for this hypothesis were: (1) that the amount of axon regeneration following chondroitinase treatment appeared insufficient by itself to mediate a major return of function; (2) behavioural improvements happen to some extent spontaneously (i.e. without chondroitinase treatment), but over a very much longer time scale than in chondroitinase treated animals; and (3) spinal cord grey matter is particularly full of CSPGs, and motor neurones and other neurones are surrounded by perineuronal nets (concentrations of CSPGs and tenascin around the cell body and dendrites of neurones).

In order to test the hypothesis further, the inventors arranged a collaboration (also reported in the Examples) to determine the effect of chondroitinase treatment in a known animal model for neuronal plasticity, the ocular dominance shift model. This model relates to plasticity in the connections from the eyes to the visual cortex.

Normally, neurones in a part of the rat visual cortex receive connections from both eyes. Most of the neurones are equally strongly driven by (i.e. have equally strong synaptic connections to) both eyes. In an animal less than 35 days old, however, if one eyelid is sutured shut to deprive it of visual experience, the neurones in this region of the cortex cease to be driven so strongly by the deprived eye and become driven much more strongly by the non-deprived eye. That is, the synaptic connections to the sutured eye are weakened, and those to the non-sutured eye are strengthened. This effect is known as ocular dominance shift, and is an example of plasticity in the cortex (Lodovichi et al., 2000). After 50 days in rats, ocular dominance shift no longer occurs after eyelid suture: this is known as the end of the critical period (Maffei et al., 1992).

The inventors proposed that digesting the glycosaminoglycan chains of chondroitin sulphate proteoglycan in the rat visual cortex with chondroitinase would restore ocular dominance plasticity in adult animals, i.e. beyond the end of the critical period, at an age when such plasticity is not seen in untreated animals. The enzyme was delivered to the visual cortex of adult rats using the same protocol used in the nigrostriatial tract experiments of Moon et al., 2001.

It was found that chondroitinase treatment to the visual cortex in adult rats allows an ocular dominance shift of a similar magnitude to that seen in young animals during the critical period.

The inventors conclude that agents, such as chondroitinase, that remove, digest, bind to and block, or prevent the synthesis of chondroitin sulphate proteoglycans (especially the glycosaminoglycan chains of CSPGs) will not only promote axon regeneration (as has previously been proposed), but will promote neuronal plasticity following CNS damage in general. Administration of such agents is expected to allow functional improvement after damage to the CNS. This has wide implications for the types of CNS damage that may be treatable by such agents.

Axon regeneration alone would not be expected to lead to significant functional recovery after most forms of CNS damage. By contrast, the teaching of the present invention that the same agents promote neuronal plasticity, leads to the expectation that these agents can be used to treat CNS damage in general.

Such an effect on plasticity could not have been predicted from the previously known effect on axon regeneration, since other factors are known to promote axon regeneration yet inhibit plasticity. Maffei et al., 1992 shows that nerve growth factor, which stimulates axon regeneration in many pathways, prevents ocular dominance shift in the rat visual cortex in the neuronal plasticity model described above. Similarly, brain-derived neurotrophic factor causes an early end to the critical period (Lodovichi et al., 2000), yet can promote axon regeneration. In light of the invention, however, some previous experimental observations can be interpreted in support of this role of CSPGs in inhibiting neuronal plasticity.

CSPGs in the mature CNS are found throughout much of the extracellular matrix, and also condensed into perineuronal nets. Perineuronal nets are dense sheaths of extracellular matrix (mostly tenascin-R and the CSPGs neurocan and aggrecan) that surround the cell body and dendrites of many neurones throughout the CNS, and almost all neurones in the spinal cord (Koppe et al., 1997). Perineuronal nets do not appear until late in development, and the time of their deposition correlates with diminution of plasticity in many systems.

In the cat visual cortex the timing of the appearance of perineuronal nets correlates precisely with the end of the critical period, and treatments that alter the timing of the critical period alter the time of deposition of perineuronal nets (Lander et al., 1997). For example, rearing animals in complete darkness delays the end of the critical period and also delays the incorporation of neurocan and aggrecan (both CSPGs) into perineuronal nets.

It seems probable, therefore, that CSPGs in perineuronal nets are responsible for restricting plasticity in the adult CNS, although the diffusely distributed CSPGs could also be involved.

Perineuronal nets may be visualised with wisteria floribunda lectin, which binds to chondroitin sulphate glycosaminoglycan chains.

### Summary of Invention

In a first aspect, the present invention provides a method of promoting neuronal plasticity in the CNS of a mammal, the method comprising administering to the CNS of the mammal an agent that reduces the inhibitory properties of chondroitin sulphate proteoglycans.

In a second aspect, the invention provides an agent that reduces the inhibitory properties of chondroitin sulphate proteoglycans, for use in a method of promoting neuronal plasticity in a mammal.

In a third aspect, the invention provides the use of an agent which reduces the inhibitory properties of chondroitin sulphate proteoglycan, in the preparation of a medicament for promoting neuronal plasticity in a mammal.

Broadly, the present invention provides in further aspects methods involved in the identification of additional agents that reduce the inhibitory properties of chondroitin sulphate proteoglycans. Following their identification, such agents can be used in accordance with the preceding aspects of the invention.

The inhibitory properties of chondroitin sulphate proteoglycans will generally be those properties that inhibit neuronal plasticity.

### Detailed Description of the Preferred Embodiments

As mentioned above, the promotion of neuronal plasticity in accordance with the invention has wide implications for the treatment of CNS damage in general. For the avoidance of doubt, the term "CNS" is intended to include the brain, the spinal cord, and neurones whose cell bodies lie within, or have a primary synapse in, the brain or spinal cord. Examples of such neurones are neurones of the cranial nerves (damage to which can e.g. cause Bell's palsy) and motor neurones that innervate the musculature and whose cell bodies are in the ventral horn of the spinal cord.

Of particular interest is the promotion of neuronal plasticity in the spinal cord and/or following spinal cord injury, especially incomplete spinal injury (i.e. spinal injury in which some intact axons project through the site of injury). The studies reported above suggest that chondroitinase can promote axonal regeneration following experimentally induced spinal cord damage in experimental animals. By contrast, the present inventors report the surprising finding that chondroitinase also promotes neuronal plasticity. This suggests that spinal cord injuries that would not previously have been considered treatable via axonal regeneration alone may now be considered treatable, via the promotion of neuronal plasticity, using chondroitinase and the other agents referred to herein.

In particular, scar tissue develops progressively following CNS damage (e.g. spinal cord injury) and after two weeks it may be difficult for axons to regenerate through the scar tissue. By contrast, it should be possible to treat spinal cord injury by the promotion of plasticity at any time after injury. Accordingly, the invention is particularly applicable to the treatment of spinal cord injuries that are at least two weeks old, especially injuries that are at least three weeks old, or at least four weeks old.

Furthermore, following spinal cord injury, damaged neurones whose axons have been cut will atrophy. This, combined with the presence of dense scar tissue, would be expected to prevent recovery of function by the promotion of axonal regeneration. By contrast, the promotion of neuronal plasticity should lead to recovery of function at any time after spinal cord injury. Accordingly, the invention is particularly applicable to the treatment of spinal cord injury which is characterised by atrophy of the neurones whose axons have been cut.

The promotion of neuronal plasticity may be the promotion of neuronal plasticity in the spinal cord and/or in the brain, especially the cortex. Sites of administration of the agent are discussed below.

The spinal cord injury may (without limitation) be injury caused by assault, accident, tumour, intervertebral disc or bone abnormality, or surgery, e.g. surgery for spinal problems and/or surgery to remove tumours.

The invention, however, also extends to the treatment of CNS damage other than spinal cord injury, particularly CNS damage of the following kinds: stroke; brain injury, including (without limitation) injury caused by assault, accident, tumour (e.g. a brain tumour or a non-brain tumour that affects the brain, such as a bony tumour of the skill that impinges on the brain) or surgery, e.g. surgery to remove tumours or to treat epilepsy; multiple sclerosis; and neurodegenerative diseases which affect the cortex, such as Alzheimer's. The invention is more particularly applicable to CNS damage affecting the cortex, since it is widely thought that promotion of plasticity will lead to functional recovery following damage to the cortex. Reference to CNS damage affecting the cortex is, however, not intended to imply the absence of damage to subcortical areas of the brain.

Multiple sclerosis leads to small lesions randomly distributed in the brain and spinal cord. While it is not thought that promotion of axon regeneration would be effective in treating multiple sclerosis, it is expected that that promotion of plasticity will lead to some recovery of function.

In Alzheimer's and other neurodegenerative diseases which affect the cortex, the damage tends to be diffuse throughout much of the cortex. Again, it is thought that promotion of plasticity is likely to lead to some recovery of function. The term "neurodegenerative disease which affects the cortex" is not intended to imply the absence of damage to other structures of the brain.

The invention is intended to exclude the treatment of nigrostriatial axotomy, as reported in Moon et al (2001). This procedure is not representative of non-experimentally inflicted CNS, especially brain, injuries (e.g. injuries inflicted by accident, assault or surgery). Preferably the invention excludes the treatment of experimentally inflicted CNS damage of non-human mammals in general, especially such damage inflicted with the aim of developing treatments for spinal cord injury by promoting axonal regeneration.

The mammal is most preferably a human. Other preferred mammals are domesticated mammals, particularly horses, cows, pigs, goats, sheep, dogs and cats. The invention is, however, generally applicable to any mammal.

The agent that reduces the inhibitory properties of chondroitin sulphate proteoglycans may be an agent that reduces the neurite-outgrowth inhibitory properties of chondroitin sulphate proteoglycans. However, neurite outgrowth is not necessarily a prerequisite of plasticity, so the agent may be one that reduces the plasticity inhibitory properties of chondroitin sulphate proteoglycans, without reducing their neurite-outgrowth inhibitory properties.

An agent which reduces the inhibitory properties of chondroitin sulphate proteoglycans may be an agent which interacts with one or more chondroitin sulphate proteoglycans (CSPGs) to inhibit their inhibitory properties, or may be an agent which eliminates (partially or completely) one or more CSPGs, or may be an agent which reduces the production of one or more CSPGs.

Preferably the agent that reduces the inhibitory properties of chondroitin sulphate proteoglycans is an agent that removes, digests, binds to and blocks, or prevents the synthesis of one or more chondroitin sulphate proteoglycans.

More preferably, the agent that reduces the inhibitory properties of chondroitin sulphate proteoglycans is an agent that removes, digests, binds to and blocks, or prevents the synthesis of the glycosaminoglycan chains of one or more chondroitin sulphate proteoglycans. Since glycosaminoglycan chains are common to all CSPGs (though differ to some extent between different CSPGs), and appear to be necessary for their inhibitory effects, they are a particularly preferred target for agents that reduce the inhibitory properties of CSPGs, as activity of agents across all the different inhibitory CSPGs is expected.

An agent which interacts with one or more chondroitin sulphate proteoglycans (CSPGs) to inhibit their inhibitory properties may be an antibody which is capable of binding one or more CSPGs, especially via the glycosaminoglycan chains of the CSPGs. NogoA is a molecule present in myelin, which inhibits axon regeneration following spinal cord injury. Anti-NogoA antibodies have been shown in Bandtlow and Schwab, 2000 to block this inhibitory property of NogoA, allowing significant functional recovery in rats. A similar effect for blocking antibodies is expected for CSPG.

Antibodies against different CSPGs and/or components of their glycosaminoglycan chains are known. For example the CS56 monoclonal antibody (Sigma) binds to a motif in the glycosaminoglycan chains of CSPGs which is upregulated following CNS injury and the antibodies CAT301, 315 and 316 are thought to bind to aggrecan (Lander et al., 1997). Moreover it is a matter of routine to prepare further antibodies (especially monoclonal antibodies) against CSPGs and/or components of their glycosaminoglycan side chains. Such known or new antibodies can be tested for their ability to reduce the inhibitory properties of CSPGs, e.g. using the techniques, particularly the behavioural techniques, described in the examples (especially beam- and grid-walking tests and footprint analysis), and/or the ability to promote neuronal plasticity, e.g. in an ocular dominance shift model such as that described in the examples.

Purified CSPGs are available as a mixture from Sigma Chemicals and Chemicon. Purified aggrecan is also available. Furthermore, single CSPGs or mixtures of CSPGs can be purified from the brain or spinal cord using well-established techniques. All would be suitable for the raising of antibodies.

The term "antibody" is used in the broadest sense and includes both monoclonal antibodies and polyclonal antibodies, as well as antibody fragments or portions such as Fv, Fab, Fab' and F(ab')2 fragments. The antibodies may chimaeric and/or humanized.

Methods for raising polyclonal antibodies from an immunising agent (e.g. in this case a CSPG) are well known. This may involve conjugating the immunising agent to an immunogenic polypeptide, such as keyhole limpet hemocyanin, serum albumin, bovine thyroglobulin, and soybean trypsin inhibitor, and/or administering it with an adjuvant such as Freund's complete adjuvant and MPL TDM adjuvant (monophosphoryl Lipid A, synthetic trehalose dicorynomycolate).

Similarly, methods for producing monoclonal antibodies are well known. See for example Goding, Monoclonal Antibodies: Principles and Practice, Academic Press, 1986, pp. 59-103, and Kozbor, J Immunol, 133:3001 (1984); Brodeur et al., Monoclonal Antibody Production Techniques and Applications, Marcel Dekker, Inc., New York, 1987, pp. 51-63. The monoclonal antibodies may also be made by recombinant DNA methods, such as those described in U.S. Patent No. 4,816,567, which may also be used to produce chimaeric and/or humanised antibodies. See for example Jones et al., Nature, 321:522-525 (1986); Riechmann et al., Nature. 332:323-327 (1988); Verhoeyen et al., Science. 239:1534-1536 (1988).

Human antibodies can also be produced using various techniques known in the art, including phage display libraries. See e.g. Hoogenboom and Winter, J. Mol. Biol, 227:381 (1991); Marks et al., J Mol Biol 222:581 (1991). The techniques of Cole et al. and Boerner et al. are also available for the preparation of human monoclonal antibodies (Cole et al., Monoclonal Antibodies and Cancer Therapy, Alan R Liss, p 77, 1985 and Boerner et al J. Immunol. 147(1):86-95 (1991)). Similarly, human antibodies can be made by introducing of human immunoglobulin loci into transgenic animals, e.g. mice. See e.g. U.S. Patent Nos 5,545,807; 5,545,806; 5,569,825; 5,625,126; 5,633,425; and 5,661,016, and Marks et al., Bio/Technology 10: 779-783 (1992); Lonberg et al., Nature 368: 856-859 (1994); Morrison, Nature 368:812-13 (1994); Fishwild et al., Nature Biotechnology 14:845-51 (1996); Neuberger, Nature Biotechnology 14:826 (1996); Lonberg and Huszar, Intern Rev Immunol 13:65-93 (1995).

Alternatively, an agent which interacts with one or more chondroitin sulphate proteoglycans (CSPGs) to inhibit their inhibitory properties may be a lectin which is capable of binding one or more CSPGs, especiallly via the glycosaminoglycan chain(s). Lectins are glycoproteins usually found in plants that bind or clump specific proteins and carbohydrates. Lectins have been used in vivo. An example of a lectin which is capable of binding one or more CSPGs is wisteria floribunda lectin. Peanut lectin binds to some CSPGs.

An agent which eliminates one or more CSPGs may for example be an enzyme capable of digesting CSPGs, especially an enzyme capable of digesting the glycosaminoglycan chains of CSPGs, e.g. chondroitinase, especially chondroitinase ABC (EC 4.2.2.4). Chondroitinase ABC is a mixture of three enzymes with activity against the three main forms of the glycosaminoglycan chains of CSPGs and is commercially available, e.g. from Seikagaku, Japan, as are other forms of chondroitinase. Moon et al. (2001) and Bradbury et al. (2002) provide proof of principle that it can be administered in vivo to mammal CNS.

Other chondroitinase enzymes include isolated components of chondroitinase ABC, including chondroitinase ABC types I and II. Their separation is disclosed in e.g. US patent 5498536. Still other chondroitinase enzymes are chondroitinase AC (EC 4.2.2.5), which is commercially available from Seikagaku and Ibex Technologies Inc. (Montreal, Quebec, Canada) and chondroitinase B, which is also commercially available from Seikagaku.

Another enzyme capable of digesting the glycosaminoglycan chains of CSPGs is hyaluronidase (EC 3.2.1.35), which is commercially available from Seikagaku and, in pharmaceutically acceptable form, from Worthington Biochemical Corporation (Lakewood, NJ, USA) or Wyeth-Ayerst (Philadelphia, Pa, USA). Other hyaluronidases may also be suitable for use in the invention, such as PH 20, hyaluronidase 1 and hyaluronidase 4, examples of which are disclosed in e.g. accession numbers S40465 (gi 631383), NP_009296 (gi 6224976) and XP_231540 (gi 27709188), respectively.

US patents 5741692, 5498536, 5763205, 5773277, 5496718, 6093563, 6054569 and 5997863 describe the purification and cloning of numerous enzymes having chondroitinase activity. See also GenBank / EMBL accession numbers: AAC83383.1 (gi 1002525) and AAC83384.1 (gi 1002527) and the related paper Tkalec et al. (2000) Appl. Environ. Microbiol. 66 (1), 29-35; and Q59288 (gi 3913237) and Brookhaven Protein Data Bank accession numbers 1CB8 and 1DBG.

The capability of an enzyme to digest the glycosaminoglycan chains of CSPGs may be assessed by assaying the ability of the enzyme, when incubated with a CSPG, to reduce its molecular weight (e.g. as determined by SDS-PAGE), generally to the molecular weight expected for the core protein alone. It may (additionally or alternatively) be assessed by assaying the ability of the enzyme to release disaccharides from the CSPG.

Also suitable for use in the invention are enzymes capable of removing the sulphate groups of CSPGs, e.g. enzymes having sulfatase activity. Numerous enzymes having this capability have been identified, such as EC 3.1.6.4, EC 3.1.6.12, EC 3.1.6.9, EC 3.1.6.10, EC 3.1.6.13, EC 3.1.6.14, EC 3.1.6.15 and EC 3.1.6.18. Chondro-4-sulfatase (EC 3.1.6.9) and chondro-6-sulfatase (EC 3.1.6.10) are commercially available from Seikagaku.

Other enzymes capable of digesting the GAG chains of CSPGs, which may also be suitable for use in the invention, are enzymes having the following activities: sulfatases (e.g. as mentioned above), endoglycosidases, exoglycosidases, hexosaminidases, galactosidases (e.g. endo-beta-N-galactosidase, commercially available from Seikagaku), glucuronidases, iduronidases, xylosidases and lysosomal enzymes.

Still other enzymes which may be capable of digesting CSPGs are enzymes of the following enzyme families, each of which has several members:
aggrecanases,
ADAMs (a disintegrin and metalloprotease),
ADAMTs (a disintegrin and metalloprotease with thrombospondin motifs), e.g.:
   ADAMTS 1 (accession number NP_008919, gi 11038654),
   ADAMTS 4 (accession number 075173, gi 12643637), also known as aggrecanase-1, and
   ADAMTS 5 (accession number NP_008969, gi 5901888), which all cleave aggrecan, cathepsins, e.g.
   cathepsin D (accession number BAC57431, gi 28436104),
   cathepsin L (accession number AA033585, gi 28194647), and
   cathepsin B (accession number NP_776456, gi 27806671),
MMPs (matrix metalloproteases), e.g.:
   MMP1 (accession number P21692, gi 116854),
   MMP2 (accession number P08253, gi 116856),
   MMP3 (accession number P08254, gi 116857),
   MMP7 (accession number P09237, gi 116861),
   MMP8 (accession number P22894, gi 116862),
   MMP9 (accession number P14780, gi 116863),
   MMP10 (accession number 055123, gi 13124340),
   MMP13 (accession number 062806, gi 5921829), and keratanases (e.g. keratanase, which is commercially available from Seikagaku).

An agent which reduces the production of one or more CSPGs may be an inhibitor of one or more of the stages involved in CSPG synthesis, e.g. an inhibitor of one or more of the enzymes responsible for CSPG synthesis, preferably one or more of the enzymes involved in the production of the glycosaminoglycan chains of CSPGs and/or their attachment to the CSPG protein moiety, e.g. one or more chondroitin sulfotransferases.

The enzymes involved in CSPG synthesis are well known from biochemical textbooks. The enzymes xylosyltransferase, galactosyltransferase I and II and glucuronosyltransferase I are involved in the attachment of a four sugar adapter stub to the core protein; N-acetylgalactosaminyltransferases and glucuronosyltransferase II are involved in the elongation of the sugar chain by addition of repeating disaccharide units; the chain is then modified by O- and N-sulfation by a variety of chondroitin sulphate sulfotransferases, of which there are at least five, sulfating in different positions. The chain is also modified by epimerisation (conversion of glucuronate to iduronate in a uronosyl epimerisation step, by C5 uronosyl epimerase) and phosphorylation (presumably by xyloside kinase).

Several chondroitin sulfotransferases have been isolated and/or cloned, e.g. chondroitin 6-O-sulfotransferase-1, chondroitin 6-O-sulfotransferase-2 chondroitin 4-O-sulfotransferase, a human uronyl 2-sulfotransferase that sulfates glucuronyl residues of chondroitin, N-acetylglucosamine-6-O-sulfotransferase and GalNAc 4-sulfotransferase (see for example Kitagawa et al (2000) J Biol Chem 275(28): 21075-80; Yamauchi et al (2000) J Biol Chem 275(12): 8975-81; Li et al (1999) Genomics 55(3): 345-7; Kobayashi et al (1999) J Biol Chem 274(15): 10474-80; Nastuk et al (1998) J Neurosci 18(18): 7167-77; Uchimura et al (1998) J Biol Chem 273(35): 22577-83; Fukuta et al (1998) Biochim Biophys Acta 1399(1): 57-61; Uchimura et al (1998) Glycobiology 8(5) 489-96; Mazany et al (1998) Biochim Biophys Acta 1407(1): 92-7; Tsutsumi et al (1998) FEBS Lett 441(2): 235-41; Fukuta et al (1997) J Biol Chem 272(51): 32321-8; Fukuta et al (1995) J Biol Chem 270(31): 18575-80; Okuda et al (2000) J Biochem (Tokyo) 128(5): 763-70; Okuda et al (2000) J Biol Chem 275(51): 40605-13). Especially preferred are the chondroitin 6-O-sulfotransferases (i.e. chondroitin 6-O-sulfotransferase-1 and chondroitin 6-O-sulfotransferase-2), which appear to be upregulated following CNS damage. Also preferred is unronyl-2-sulfotransferase (Kobayashi et al. (1999) J Biol Chem 274(15):10474-80), which sulfates chondroitin sulfate chains at a second position after previous 6-sulfation. This doubly sulfated chondroitin sulfate (known as chondroitin sulfate D) may be particularly inhibitory, and is specifically increased in the adult compared to immature CNS. There is also evidence that the enzyme is upregulated after injury.

Inhibition of sulfation may be assessed by incubating CSPG-producing cells (such as oligodendrocyte precursors) with radioactive sulphate under conditions suitable for CSPG production, and assaying the incorporation of radioactive sulphate into a CSPG fraction obtained from the cells (e.g. a CSPG-containing fraction separated biochemically on an ion exchange column or immunologically on an affinity column, e.g. using antibodies to the core protein).

Several potentially relevant four sugar adapter stub attachment enzymes and sugar chain elongation enzymes have been isolated and/or their mRNAs identified and deposited. See Table 1.

mRNA for a putative C5 uronosyl epimerase has also been identified and deposited, under accession number XM 035390 (gi number 14749930).

All accession and gi numbers used herein refer to the GenBank / EMBL database.

**Table 1**

| Enzyme | mRNA sequence accession number | Protein sequence accession number | gi number |
|---|---|---|---|
| (Attachment of four sugar adapter stub to core protein) | | | |
| Xylosyltransferase I | XM 085432 | | 18585499 |
| Xylosyltransferase II | XM 037916 AJ 277442 | | 14773240 11322267 |
| Galactosyltransferase I | AB 028600 BC 007317 NM 007255 | | 5738914 13938367 6005951 |
| Galactosyltransferase II | NM 080605 AY 050570 | | 18079318 16024927 |
| Glucuronosyltransferase I | NM 012200 XM 071531 BC 007906 | | 12408653 18605106 14043939 |
| (Elongation of sugar chain) | | | |
| N-acetylgalactosaminyltransferase | XM 006665 | | 13649549 |
| N-acetylgalactosaminyltransferase 1 | XM 041524 | | 14762496 |
| N-acetylgalactosaminyltransferase 2 | XM 001319 | | 18545231 |
| N-acetylgalactosaminyltransferase 3 | XM 002282 | | 18552455 |
| N-acetylgalactosaminyltransferase 4 | NM 003774 | | 13124892 |
| N-acetylgalactosaminyltransferase 5 | XM 050509 | | 18599704 |
| N-acetylgalactosaminyltransferase 6 | NM 007210 | | 13124893 |
| N-acetylgalactosaminyltransferase 7 | XM 003527 XM 084139 NM 054110 AY 035399 | | 12230727 18555932 16905368 16756124 |
| N-acetylgalactosaminyltransferase 8 | NM 017417 | | 8393411 |
| N-acetylgalactosaminyltransferase 9 | XM 039991 | | 18560247 |
| Glucuronosyltransferase II | | Q9NPZ5 | 14285363 |

An inhibitor of one or more of the enzymes responsible for CSPG synthesis may for example be a blocking antibody against one of the above-identified synthetic enzymes or a blocking antibody against the substrate of such an enzyme. Other agents include agents modelled on the substrates of the CSPG synthetic enzymes, which are capable of blocking synthesis of CSPGs. Such agents include β-D-xylosides, which prevent the attachment of glycosaminoglycan chains to the protein moieties of CSPGs, and which can be administered in vivo (Zuo et al., 1998).

An agent that reduces the production of one or more CSPGs may be an antisense nucleic acid molecule which is capable of blocking the translation of a CSPG core protein, or a dsRNA or siRNA molecule which is capable of suppressing expression of a CSPG core protein by RNAi. Nucleic acid sequences for several CSPG core proteins are known. See for example accession numbers XM 009327 and NM 004386 (gi numbers 14766861 and 4758083) for neurocan, accession numbers XM 031288 and NM 013227 (gi numbers 14753428 and 6695993) for aggrecan, accession numbers BC 022938 and XM 044090 (gi numbers 18605563 and 18549315) for brevican and accession numbers NM 004385 and X 15998 (gi numbers 4758081 and 37662) for versican.

Antisense technology has reached an advanced state, and delivery to cells may be accomplished by for example incorporation into a viral vector, linkage to a cell-permeant moiety, e.g. peptide, or by the use of a morpholino antisense molecule.

Short antisense oligonucleotides can be imported into cells where they act as inhibitors, despite their low intracellular concentrations caused by their restricted uptake by the cell membrane. (Zamecnik et al., Proc. Natl. Acad. Sci. USA 83, 4143-4146 [1986]). The oligonucleotides can be modified to enhance their uptake, e.g. by substituting their negatively charged phosphodiester groups by uncharged groups. In vivo gene transfer techniques include transfection with viral (typically retroviral) vectors and viral coat protein-liposome mediated transfection (Dzau et al., Trends in Biotechnology _11, 205-210 [1993]). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, proteins that target intracellular localization and enhance intracellular half-life. The technique of receptor-mediated endocytosis is described, for example, by Wu et al., J Biol Chem 262, 4429-4432 (1987); and Wagner et al., Proc. Natl Acad. Sci USA 87, 3410-3414 (1990). For review of gene marking and gene therapy protocols see Anderson et al., Science 256 808-813 (1992).

An alternative to anti-sense is to use a copy of all or part of the target gene inserted in sense, that is the same, orientation as the target gene, to achieve reduction in expression of the target gene by co-suppression; Angell & Baulcombe (1997) The EMBO Journal 16,12:3675-3684; and Voinnet & Baulcombe (1997) Nature 389: pg 553). Double stranded RNA (dsRNA) has been found to be even more effective in gene silencing than both sense or antisense strands alone (Fire A. et al Nature, Vol 391, (1998)). dsRNA mediated silencing is gene specific and is often termed RNA interference (RNAi).

RNA interference is a two step process. First, dsRNA is cleaved within the cell to yield short interfering RNAs (siRNAs) of about 21-23nt length with 5' terminal phosphate and 3' short overhangs (∼2nt) The siRNAs target the corresponding mRNA sequence specifically for destruction (Zamore P.D. Nature Structural Biology, 8, 9, 746-750, (2001)

RNAi may be also be efficiently induced using chemically synthesized siRNA duplexes of the same structure with 3'-overhang ends (Zamore PD et al Cell, 101, 25-33, (2000)). Synthetic siRNA duplexes have been shown to specifically suppress expression of endogenous and heterologeous genes in a wide range of mammalian cell lines (Elbashir SM. et al. Nature, 411, 494-498, (2001)).

See also Fire (1999) Trends Genet. 15: 358-363, Sharp (2001) Genes Dev. 15: 485-490, Hammond et al. (2001) Nature Rev. Genes 2: 1110-1119 and Tuschl (2001) Chem. Biochem. 2: 239-245.

Such antisense or RNAi inhibition may also be directed against the enzymes involved in the synthesis of CSPG glycosaminoglycan chains.

An agent that reduces the production of one or more CSPGs may be an agent that kills one or more of the non-neuronal cell types that produces CSPGs, such as oligodendrocyte precursors. This has been accomplished for oligodendrocyte precursors using cytosine arabinoside (Rhodes et al., 2000), an anti-mitotic agent.

Proteoglycans in the brain and spinal cord, including CSPGs, are held in place by their interactions with other matrix molecules, particularly hyaluronan. The blockage, elimination or reduction of production of hyaluronan will therefore remove the attachment of CSPGs to the tissue, allowing them to diffuse away, removing their inhibitory effect.

Accordingly, an agent which reduces the inhibitory properties of chondroitin sulphate proteoglycans may be an agent which interacts with hyaluronan to inhibit its ability to bind to CSPGs, or may be an agent which eliminates (partially or completely) hyaluronan, or may be an agent which reduces the production of hyaluronan, or may be an agent which blocks, destroys or reduces the synthesis of a receptor by which hyaluronan (and hence CSPGs) is anchored to the surface of cells in the CNS.

The above description of blockage, elimination and reduction of production of CSPGs applies mutatis mutandis to the blockage, elimination and reduction of production of hyaluronan and to the blockage, elimination and reduction of production of hyaluronan receptors. In this context, hyaluronidase (which digests hyaluronan) may be used to eliminate hyaluronan; hyaluronan synthases 1, 2 and 3 (which are involved in the synthesis of hyaluronan) may be targets for reducing the production of hyaluronan; and the hyaluronan receptors CD44 (Bosworth et al. (1991) Mol Immunol 28(10):1131-5), Lyve 1 (Banerji et al. (1999) J Cell Biol 144(4):789-801), RHAMM (Wang et al. (1996) Gene 174(2):299-306; Spicer et al. (1995) Genomics 30(1) 115-7), layilin (Bono et al. (2001) Mol Biol Cell 12(4):891-900) and ICAM-1 may be targets for reducing the ability of hyaluronan to anchor CSPGs to CNS cells.

It is also thought that link proteins (such as cartilage link protein (Dudhia et al. (1994) Biochem J 303(Pt 1):329-33) and BRAL-1 (Hirakawa et al. (2000) Biochem Biophys Rec Commun 276(3):982-9)) may be involved in the attachment of CSPGs to the cell surface. The above description of blockage, elimination and reduction of production of CSPGs applies mutatis mutandis to the blockage, elimination and reduction of production of link proteins.

An agent that reduces the inhibitory properties of CSPGs may be a sugar molecule that binds to the GAG chains of one or more CSPGs or, more preferably a sugar molecule that mimics the GAG chains of one or more CSPGs and/or hyaluronan and thereby acts as a competitive antagonist. The structures of the GAG chains of CSPGs and of hyaluronan are well known. Particularly preferred sugar molecules comprise sulfated, repeating disaccharides of glucuronic acid and N-acetyl galactosamine.

Furthermore, the CSPGs, the glycosaminoglycan chains thereof, CSPG synthetic enzymes, and/or the substrates of those enzymes (i.e. intermediates in the synthesis of CSPGs) may be used to identify leads for the development of further agents which reduce the inhibitory properties of chondroitin sulphate proteoglycans. In particular, a candidate lead compound may be tested for binding to a CSPG, CSPG synthetic enzyme or CSPG synthetic enzyme substrate using standard techniques. Compounds which bind may be tested for the property of reducing the inhibitory properties of CSPGs and/or promoting neuronal plasticity, e.g. using the techniques described in the examples (especially the behavioural techniques and/or the ocular dominance shift model).

Especially prior to in vivo testing, a compound which binds may be tested for the property of reducing neurite-outgrowth inhibition following CNS damage, for example in the in vitro models used in the works referred to above and in the examples in relation to the work on spinal cord injury.

Following the identification of a lead compound which has the property of reducing neurite-outgrowth inhibition following CNS damage, the lead compound may be subjected to optimisation for desirable pharmaceutical properties, e.g. by the production and screening of mimetics of the lead compound.

The designing of mimetics to a known pharmaceutically active compound is a known approach to the development of pharmaceuticals based on a "lead" compound. This might be desirable where the active compound is difficult or expensive to synthesise or where it is unsuitable for a particular method of administration, eg peptides are unsuitable active agents for oral compositions as they tend to be quickly degraded by proteases in the alimentary canal. Mimetic design, synthesis and testing is generally used to avoid randomly screening large number of molecules for a target property.

There are several steps commonly taken in the design of a mimetic from a compound having a given target property. Firstly, the particular parts of the compound that are critical and/or important in determining the target property are determined. In the case of a peptide, this can be done by systematically varying the amino acid residues in the peptide, eg by substituting each residue in turn. These parts or residues constituting the active region of the compound are known as its "pharmacophore".

Once the pharmacophore has been found, its structure may be modelled to according its physical properties, eg stereochemistry, bonding, size and/or charge, using data from a range of sources, eg spectroscopic techniques, X-ray diffraction data and NMR. Computational analysis, similarity mapping (which models the charge and/or volume of a pharmacophore, rather than the bonding between atoms) and other techniques can be used in this modelling process.

In a variant of this approach, the three-dimensional structure of the ligand and its binding partner are modelled. This can be especially useful where the ligand and/or binding partner change conformation on binding, allowing the model to take account of this in the design of the mimetic.

A template molecule is then selected onto which chemical groups which mimic the pharmacophore can be grafted. The template molecule and the chemical groups grafted on to it can conveniently be selected so that the mimetic is easy to synthesise, is likely to be pharmacologically acceptable, and does not degrade *in vivo,* while retaining the biological activity of the lead compound. The mimetic or mimetics found by this approach can then be screened to see whether they have the target property, or to what extent they exhibit it. Further optimisation or modification can then be carried out to arrive at one or more final mimetics for *in vivo* or clinical testing.

Accordingly, the present invention provides, in a further aspect, a method of identifying an agent useful in promoting neuronal plasticity and/or for the treatment of CNS damage other than spinal cord injury, the method comprising the steps of:
(a) bringing into contact (i) a candidate agent and (ii) a CSPG, a CSPG glycosaminoglycan chain, a CSPG synthetic enzyme or a CSPG synthetic enzyme substrate;
(b) determining binding between (i) and (ii); and
(c) following a positive determination of binding, assaying the candidate agent for the ability to promote neuronal plasticity and/or to promote functional recovery following CNS damage; and optionally
(d) optimising the candidate agent for in vivo use by further steps including generating mimetics of the candidate agent and repeating steps (a) and (b) and/or repeating step (c).

The invention further provides a method of identifying an agent useful in promoting neuronal plasticity, the method comprising the steps of:
(a) bringing into contact (i) a candidate agent and (ii) CSPG or a CSPG glycosaminoglycan chain;
(b) determining the ability of (i) to digest (ii); and
(c) following a positive determination of digestion, assaying the candidate agent for the ability to promote neuronal plasticity and/or to promote functional recovery following CNS damage; and optionally
(d) optimising the candidate agent for in vivo use by further steps including generating mimetics of the candidate agent and repeating steps (a) and (b) and/or repeating step (c) with the mimetic as the candidate agent.

Preferred features of these methods are as defined above.

The methods may include a step preceding step (c) of assaying the candidate agent for the ability to reduce the neurite-outgrowth and/or axon regeneration inhibitory properties of CSPGs following CNS damage.

In a still further aspect, the invention provides the use of a CSPG, a CSPG glycosaminglycan chain, a CSPG synthetic enzyme or a CSPG synthetic enzyme substrate in the identification of an agent useful in promoting neuronal plasticity.

In one preferred embodiment, the candidate agent may be a mimetic of a β-D-xyloside. β-D-xylosides are known to prevent CSPG synthesis, by competing with the CSPG protein for binding of the glycosaminoglycan chain, so are a particularly suitable starting point for the identification of further agents.

Following identification of an agent useful in promoting neuronal plasticity, the agent may be formulated with one or more conventional excipients acceptable for pharmaceutical or veterinary use. Such a formulation may be administered to promote neuronal plasticity in a mammal.

Administration may be by any conventional route, in particular any route capable of delivering the agent to the CNS, across the blood brain barrier. The preferred route of administration will be by direct administration to the CNS, e.g. infusion via cannula or injection. Such administration may be directly into the site of injury, into neighbouring tissues, or into the cerebrospinal fluid. See for example the administration of chondroitinase ABC to rat brain as described in Moon et al (2001) and Bradbury et al (2002).

Of particular interest is administration to the spinal cord at a site above and/or below the site of a spinal cord injury. Two thirds of spinal cord injuries involve incomplete transections of the cord, with some axons surviving through the injury. In half these incomplete cases, there is useful neurological function below the lesion. Enhancing plasticity below the lesion should allow the surviving axons to innervate neurones in the cord more widely, and therefore should lead to return of function. Similarly, it is proposed that enhancing plasticity above the level of the lesion will allow cut axons to make new connections to neurones whose axons still survive and project below the lesion, thereby carrying some function to the cord below the lesion.

Also of interest is administration to the brain (especially the cortex) in the case of spinal cord injury. It is proposed that enhancing plasticity in the brain could allow patients to make better use of their surviving connections in the cord. Indeed, plasticity in the cortex can be induced temporarily by repetitive magnetic stimulation, and there is evidence that this may be helpful to patients with incomplete injuries.

Preferred CSPGs in relation to all aspects of the invention are NG2, versican (V0, V1 and V2 forms), neurocan, brevican, phosphacan and aggrecan, all of which are present at high levels following CNS damage, and all of which are known to be inhibitory to axon regeneration. Neurocan, aggrecan, brevican and phosphocan have been reported in perineuronal nets and are preferred targets for inhibition to promote neuronal plasticity.

The foregoing description of the identification of agents applies mutatis mutandis to hyaluronan and its receptors and to link proteins.

Although the foregoing invention has been described in some detail by way of illustration and example for purposes of clarity and understanding, it will be readily apparent to those of ordinary skill in the art in light of the teachings of this invention that certain changes and modifications may be made thereto without departing from the spirit or scope of the appended claims. All cited documents are incorporated herein by reference in their entirety and for all purposes.

### Example 1 - Chondroitinase ABC promotes axon regeneration and functional recovery following spinal cord injury.

### Summary

The inability of axons to regenerate in the mammalian central nervous system can lead to permanent paralysis after a spinal cord injury. At CNS injury sites a glial scar develops, containing a variety of extracellular matrix molecules including chondroitin sulphate proteoglycans (CSPGs) (Fawcett and Asher, 1999). Regenerating axons stop at these CSPG-rich regions (Davies et al., 1999) and many CSPGs have been shown to be inhibitory to axon growth in vitro (McKeon et al., 1991; Fidler et al., 1999; Niederost et al., 1999). Removing CSPG glycosaminoglycan (GAG) chains attenuates CSPG inhibitory activity in vitro (Smith-Thomas et al., 1994; McKeon et al., 1995; Zuo et al., 1998) and in vivo Moon et al., 2001). To test the functional effects of degrading chondroitin sulphate GAGs (CS-GAGs) after spinal cord injury, chondroitinase ABC (ChABC) was delivered to the lesioned dorsal columns of adult rats. Intrathecal treatment with ChABC degraded CS-GAG chains at the injury site, up-regulated a regeneration-associated protein in injured neurons and promoted regeneration of both ascending sensory projections and descending corticospinal tract axons.

ChABC treatment also restored postsynaptic activity below the lesion following electrical stimulation of corticospinal neurons. Finally, ChABC treatment promoted functional recovery of some locomotor and proprioceptive behaviours.

### Results and Discussion

Adult rats received a cervical level 4 (C4) dorsal column crush lesion and bolus intrathecal infusions of protease-free ChABC or a control solution over 10 days.

First, the effectiveness of this treatment in degrading CS-GAG chains at a spinal cord injury site was investigated using the antibody 2B6. This identifies an epitope created on CSPG core proteins after ChABC digestion and does not recognise intact CS-GAG (Moon et al., 2001). In unlesioned controls and lesioned animals which received control infusions, no 2B6 immunostaining was apparent. However, in vitro incubation of adjacent lesioned tissue sections with ChABC revealed an abundance of 2B6 immunoreactivity throughout the entire cord section, confirming that CSPGs are present in and around the injury site following a dorsal column lesion. In lesioned rats treated in vivo with ChABC, there was intense immunoreactivity for 2B6 around the lesion site and in white matter tracts extending for at least 4 mm rostrally and caudally. Thus, *in vivo* delivery of ChABC successfully removed CS-GAG chains from CSPGs at and around the site of a spinal cord injury and in white matter tracts where dorsal column axons project.

ChABC treatment effects were assessed using the expression of the axon growth-associated protein GAP-43 in sensory neurons. GAP-43 is up-regulated in this population following peripheral nerve injury, where regeneration occurs, but not following central injury (Chong et al., 1994), where regeneration does not normally occur (Bradbury et al. 2000b), and thus is associated with a regenerative state. GAP-43 expression was assessed in C5 and C6 dorsal root ganglia (DRG). In sham-operated controls, GAP-43 protein was present in approximately 25% of small (< 40 µm) and 6% of large (> 40 µm) diameter neurons, and levels were unchanged 8 weeks after dorsal column lesions with control infusions. However, GAP-43 expression in ChABC-treated lesioned animals, compared to controls, was markedly increased in large-diameter cells (from 5.9 ± 1.4% to 22.4 ± 2.3%, data are mean ± sem, p<0.001, one-way ANOVA), which have axons that project through the lesion site. Thus degrading CS-GAG components of inhibitory CSPGs at the lesion site allows for the up-regulation of GAP-43 in axotomised neurons, indicating a propensity for these cells to regenerate.

To ascertain whether ChABC promoted regeneration of injured ascending dorsal column axons at 8 weeks post-lesion, the forelimb dorsal column projection was labelled using cholera toxin beta subunit (CTB) tracing. Following lesion and control infusion, retraction of the CTB-labelled fibre bundle had occurred, with few fibres approaching the lesion site and none entering lesioned tissue. In contrast, in all lesioned animals that received ChABC infusions, there was less fibre retraction, with thick fibre bundles observed approaching the lesion site. Strikingly, many axons traversed lesioned tissue in ChABC-treated rats, with bundles of regenerating axons observed growing around cavities and within the lesion epicentre. Growth cone-like endings were apparent on axon tips in ChABC-infused rats, indicating they were indeed regenerating. Bundles of axons were observed to grow for up to 2 mm through lesioned tissue and many individual fibres were apparent at 4 mm past the lesion site, indicating robust regeneration of lesioned ascending dorsal column axons.

Regeneration of the corticospinal tract (CST), the major descending pathway that runs in the dorsal columns, was also investigated. To confirm complete lesion of the CST, PKC-γ (a marker of the CST system, Mori et al., 1990) immunostaining was performed in lumbar cord. In unlesioned cord PKC-y immunoreactivity was observed in lamina II of the dorsal horn and in the CST running in the ventral part of the dorsal columns. However, in all lesioned animals no PKC-γ immunoreactivity was observed in the CST at lumbar levels, confirming complete transection of this tract. To assess axon regeneration CST axons were labelled using the anterograde tracer biotinylated dextran amine (BDA) injected into the motor cortex. In unlesioned animals a thick fibre bundle was observed in cervical spinal cord. At ten weeks post-injury, CST axons in lesioned rats treated with vehicle did not approach the lesion, indicating CST retraction following dorsal column injury (Hill et al., 2001), and no fibres were seen beyond the lesion site. However, ChABC treatment prevented CST retraction and promoted regeneration, with significantly more fibres seen up to and below the lesion than in vehicle-treated animals (P < 0.001, two-way ANOVA). Data were fibre counts above and below the lesion, expressed as percentages (± sem) of axons counted at 4 mm above the lesion. Some regenerating fibres in ChABC treated animals sent axon collaterals from white matter into gray matter, indicating terminal arborization and possible synaptic interactions of regenerating axons.

Terminal electrophysiological experiments were then performed to determine whether regenerated CST axons established any functional connections. In anaesthetised control animals, A-fibre strength (100µA, 200µs) electrical stimuli applied to motor cortex evoked large cord dorsum potentials, as previously described (Wall and Lidierth, 1997), with an average latency of 3.9 ± 0.4 ms at C4. Acute dorsal column lesions largely abolished these recorded potentials, even by 1 mm below the lesion. The small remaining response presumably represents post-synaptic activity induced by the descending motor pathways not running in the dorsal columns. In vehicle-treated animals, studied 13-17 days after dorsal column lesions, cortical evoked potentials were present above the lesion, but, as with acute lesions, were largely abolished below the lesion (P < 0.05, two-way ANOVA). In contrast, the ChABC-treated animals showed clear evoked responses, recorded up to 7 mm below the lesion, averaging about 40% of the size recorded above the lesion, which were not significantly different from responses in sham preparations (p > 0.1, two-way ANOVA). These responses had a normal configuration, but were delayed compared to controls (mean latency of 18.2 ± 2.6 ms, p < 0.005, one-way ANOVA), consistent with poor re-myelination of regenerating axons (Ramer et al., 2000). Similar responses were seen in all ChABC-treated animals, and were abolished by acute resection of the dorsal columns, very strong evidence that they represented newly formed connections of regenerated CST axons.

Dorsal column projections are important for fine discriminative touch and proprioception and, together with local spinal reflexes, for locomotion and skilled motor function. Thus, rats were assessed on a number of behavioural tasks of forelimb function requiring integration of sensorimotor skills. An adhesive tape-removal task assessed both sensory (awareness of the tape) and motor (ability to remove the tape) function. Unlesioned sham controls rapidly sensed the presence of the tape and removed it. Lesioned rats treated with either vehicle or ChABC were severely impaired on both aspects of the task, compared to sham controls (at 6 weeks post-lesion latencies [s] were 7.4±1.4 and 3.8±0.5 [sham], 54.9±3.1 and 48.0±4.4 [lesion plus vehicle], 41.7±8.3 and 40.5±11.1 [lesion plus ChABC] for sense and remove respectively, p < 0.001, two-way ANOVA). These impairments persisted for the entire six weeks of testing. The failure of ChABC treatment to promote recovery in this task is consistent with the fact that sensory axons did not regenerate as far as the hindbrain sensory nuclei.

In two locomotor tasks the number of forelimb footfalls were recorded when rats crossed a narrow beam or grid. In both tasks unlesioned sham controls made few, if any, footfalls. All vehicle-treated lesioned rats were severely and significantly impaired on both tasks for the entire testing period, despite some spontaneous recovery over time (Murray 1997) (p < 0.001, two-way ANOVA; at 6 weeks post-lesion footfalls had increased, compared to sham controls, from 0.0 ± 0.0 to 21.1 ± 4.2 and 0.8 ± 0.5 to 8.8 ± 1.9 on the beam and grid, respectively). ChABC treatment resulted in a striking recovery of function on both tasks, with improvements seen from 2 weeks post-lesion on the beam and 1 week post-lesion on the grid, lasting for the entire testing period (not significantly different from sham controls, p > 0.1, two-way ANOVA; at 6 weeks post-lesion footfalls were 2.6 ± 0.8 and 1.6 ± 0.7 on the beam and grid, respectively).

The walking patterns of rats were also assessed by analysing footprint spacing during continuous locomotion. Lesioned rats treated with vehicle were found to take significantly shorter and wider strides than sham controls (at 6 weeks post-lesion, length decreased, compared to controls, from 148.9 ± 4.8 to 118.1 ±6.3 mm and width increased from 12.5 ± 1.9 to 25.2 ± 3.7 mm, P < 0.02, one-way ANOVA). However, these changes were largely prevented in ChABC-treated animals, where stride length and width did not significantly differ from sham controls (length and width were 137.1 ± 11.0 and 15.4 ± 3.6 mm, respectively, P > 0.1, one-way ANOVA).

These results demonstrate that ChABC promotes regeneration and restoration of function following spinal cord injury. These effects were apparent using anatomical, electrophysiological and behavioural outcome measures. A variety of inhibitory CSPGs, including neurocan, phosphacan, brevican and NG2, are up-regulated at CNS injury sites (Levine, 1994; Yamada et al., 1997; McKeon et el., 1999; Asher et al., 2000) and we have demonstrated that CS-GAG chains are responsible for a significant part of their inhibitory properties.

Only partial regeneration of lesioned axons was induced, presumably due to the presence of other inhibitory mechanisms in the lesioned spinal cord environment (Bregman et al., 1995; Pasterkamp et al., 2001). However, even with limited regeneration of dorsal column axons, we have observed very clear recovery of function following ChABC treatment, for which there are several possible anatomical substrates. First, the limited re-growth of sensory and CST motor axons seen here might, via new local segmental connections, account for the behavioural recovery. Second, CSPGs may function in the adult to inhibit intact pathways from sprouting, and removal of this inhibition by ChABC may result in beneficial compensatory sprouting mechanisms, or plasticity, similar to those observed after IN-1 treatment (Thallmair et al., 1998).

### Methods

### Spinal cord injury and ChABC treatment:

Adult male Wistar rats received a bilateral lesion to the dorsal columns as previously described (Bradbury et al., 1999), performed at spinal level C4. Concurrently, a silastic tube was inserted intrathecally via the atlanto occipital membrane to lie just rostral to the lesion site. The other end of the catheter was externalised in order to deliver bolus injections of high-purity, protease-free Chondroitinase ABC (ChABC, Seikagaku Corporation). Immediately after the lesion, 6 µl ChABC (0.1 U/ml) was injected followed by a 6 µl saline flush (Les + ChABC, n=17). A further group (Les + vehicle, n=21) received the spinal cord lesion and were treated with either saline or a control enzyme, Penicillinase (Sigma, same µg protein delivered). ChABC or control solution was delivered on alternate days for 10 days postlesion. Control animals (n=20) received sham surgery.

Rats were either perfused at 2 weeks post-lesion to check effectiveness of treatment in removing CS-GAG (n=4 per group) or tested behaviourally over 6 weeks (n= 5, 9, 8, ChABC, vehicle, sham, respectively) then perfused at 8 weeks post-lesion for anatomical analysis of ascending systems (n=4 per group). Further rats were perfused at 10 weeks post-lesion for analysis of CST regeneration (n=4 per group) or used for terminal electrophysiology experiments at 14-17 days post-lesion (n=4 per group).

### 2B6 immunostaining to confirm CS-GAG digestion:

Tissue processing was as previously described (Bradbury et al., 1999). To ascertain the effectiveness of ChABC delivery, parasagittal sections (20 µm) of cervical spinal cord were immunostained using monoclonal antibody 2B6 (Seikagaku Corporation, 1:1000), with tyramide signal amplification (NEN). As an over-digestion control, tissue sections from lesioned cords treated with vehicle in vivo were incubated in vitro with ChABC (1:50,2 h, 37°C) prior to immunostaining. This gave an indication of the maximal effects on CS-GAG chain digestion (and resulting 2B6 immunostaining) that could be achieved with ChABC treatment. Tissue from all experimental conditions was processed in parallel.

### GAP-43 analysis in dorsal root ganglion neurons:

Sections of C5 and C6 DRG (10 µm) were double-immunostained for βIII tubulin (Promega, 1:1000), to identify all neurons, and for the growth-associated protein GAP-43 (gift from G. Wilkin, 1 :2000), using AMCA- and TRITC-conjugated secondary antibodies. For each group images of immunostaining were captured for 4 sections/animal and the percentage of cells positive for GAP-43 was determined for cells smaller than and greater than 40 µm in diameter.

### Axon tract tracing in the spinal cord:

Ascending axons that project in the dorsal columns were labelled using the cholera toxin β subunit (CTB) tracer as previously described (Bradbury et al., 1999), injected into the left median nerve to label forelimb sensory afferents. Longitudinal sections (20 µm) were immunostained for glial fibrillary acidic protein in order to identify the lesion site, and for cm, to identify the labelled dorsal column axons (Bradbury et al., 1999).

For analysis of descending CST axons, rats were anaesthetised with sodium pentobarbital (40 mg/kg) and placed in a stereotaxic apparatus. A portion of skull over the left primary motor cortex was removed, the dura mater incised and biotinylated dextran amine (BDA, Molecular Probes Inc.) was injected into the left primary motor cortex. Each animal received 4 evenly spaced 1 µl injections of BDA (10% in saline) 1 mm below the dorsal surface of the brain via a Hamilton syringe. Gelfoam was then placed over the exposed brain and the scalp sutured closed. Rats were left for a further 2 weeks before perfusing. BDA-labelled fibres were visualized in parasaggital spinal cord sections (20 µm) with extra-avidin conjugated to FITC. All BDA-labelled fibres observed within a 1 mm square grid were counted at measured intervals from 4 mm above to 5 mm below the lesion site by an experimenter blinded to treatment. Due to variability in labelling, axon numbers at the different points were calculated as a percentage of the fibres seen at 4 mm above the lesion, where the CST was intact.

In order to verify a complete CST lesion, transverse sections from the lumbar spinal cord (20 µm) were immunostained with an antibody against the y subunit of protein kinase-C (PKCγ, Santa Cruz, 1:1000), visualized with TRITC. PKCγ is expressed by afferent terminals and cell bodies within lamina II inner of the dorsal horn and by the CST projecting in the dorsal columns (Mori et al., 1990). Thus, PKCγ expression in the lumbar dorsal horn should not be affected by cervical dorsal column lesion, whereas CST immunoreactivity should be absent following CST transection.

### Electrophysiology:

In terminal electrophysiological experiments, the sensory motor cortex and cervical spinal cord was exposed in urethane-anaesthetised (1.5 g/ kg) rats that had undergone sham or dorsal column lesion surgery (treated with either vehicle or ChABC) 13-17 days previously. Cortical evoked potentials were elicited by electrical stimulation (5 square wave pulses at 400Hz, 100 pA, 200µs, delivered every 2 seconds) of the left sensory-motor cortex using a 0.5 mm concentric needle electrode lowered 1 mm into the cortex. At the beginning of each experiment the optimal stimulation site was mapped, being located 1-2 mm lateral and 1 mm rostrocaudal from Bregma. Post-synaptic potentials evoked by the cortical stimuli were recorded with a silver ball electrode placed medially on the contra-lateral cord surface. At each recording site (1 segment above, and 1-7 mm below the lesion at C4), 64 responses were averaged and stored for off-line analysis of response magnitude (area under curve) and latency. Data were normalised to the size of the rostral recording.

### Behavioural assessment:

Prior to any surgery rats were extensively handled and habituated to the various tasks. Animals were then tested on each task at two time points before surgery to obtain baseline scores and once a week for 6 weeks post-lesion. No differences were observed between right and left forepaw scores so the two were averaged. Experimenters were blind to treatment.

An adhesive tape removal test (adapted from Thallmair et al., 1998) produced separate scores for sensory and motor behaviour. A rectangle of adhesive tape (0.3" x 1") was placed on the forepaw and the time taken to sense the presence of the tape (indicated by paw shake) was determined. For animals that sensed the tape before the 60 sec cut off, the time taken to remove the tape was also scored. At each post-lesion time point scores for two trials were averaged.

Rats were tested on two locomotor tasks which require sensorimotor integration (sensory feedback and motor coordination) for accurate performance (adapted from Kunkel-Bagden et al., 1993). Rats were trained to cross a narrow metal beam (1¼" x 36") and a wire grid (12" x 36" with 1" x 1" grid squares) by placing a dark escape box at one end. The number of forepaw footfalls were recorded while navigating across the beam (determined by misplacement of the foot on the beam resulting in a slip over the side) and grid (determined by failure to grasp a rung resulting in a drop of the foot below the plane of the grid). The total scores for two runs at each time point were calculated.

In a footprint analysis (also adapted from Kunkel-Bagden et al., 1993), rat forepaws were inked to record walking patterns as rats crossed a wooden runway (4" x 36") covered in white paper.

Pre-training the rats using the dark escape box allowed the assessment of footprints made during continuous locomotion. For each rat measurements of forepaw stride length and stride width were calculated from 6 strides (3 left and 3 right) at each postlesion time point.

### Example 2 - Chondroitinase ABC treatment leads to ocular dominance shift in adult rats.

In collaboration with workers in Pisa, experiments have been performed to test whether the chondroitinase treatment developed by Moon et al. could restore ocular dominance plasticity to the adult cortex.

In the binocular area of the rat visula cortex, most neurones are stimulated equally by both eyes. From birth up to day 35, suturing one eye closed leads to most cortical neurones receiving their strongest input from the open eye, with few neurones driven by the closed eye. This change is known as ocular dominance shift. After day 35, the effect of eye closure on ocular dominance gradually diminishes, with no effect of eye closure after day 50.

The investigators examined the distribution of CSPGs and tenascin-R during this process. The condensation of neurocan and tenascin-R into perineuronal nets begins as the critical period starts to end, and formation of perineuronal nets visualised with wisteria lectin also occurs at this time. Various previous investigations have shown perineuronal net formation in various parts of the brain at this time, and in particular that three antibodies that probably bind to aggrecan (CAT301, 315, 316: Lander et al. 1997) reveal perineuronal nets in the cat visual cortex co-incident with the end of the critical period.

The investigators hypothesised that the laying down of CSPGs in the cortex, and particularly their concentration in perineuronal nets, might be instrumental in restricting cortical plasticity and preventing ocular dominance shift after eye closure after the critical period. They therefore injected chondroitinase ABC into the visual cortex of adult rats, then sutured one eye closed. 7 days later, they recorded from the cortex to assess ocular dominance. They found that the ocular dominance of neurones in the visual cortex had shifted towards the non-deprived eye, just as in young animals subjected to eye suture during the critical period. They concluded that the laying down of proteoglycans in the cortex is a mechanism for the ending of the critical period, and for the restriction of cortical plasticity.

Since proteoglycans and proteoglycans in diffuse distribution and in perineuronal nets are found throughout the cortex, and since cortical plasticity in general becomes restricted compared with newborn animals, it is probable that restriction of plasticity by proteoglycans is a general mechanism in the cortex, other parts of the brain and spinal cord. It is therefore proposed that treatments that remove the inhibitory effects of proteoglycans would stimulate plasticity in all parts of the central nervous system.

The dose and method of application were as described in Moon et al., 2001. Other procedures for the handling of the rats were as described in Lodovichi et al., 2000 and Maffei et al., 1992.

### References:

Asher RA, Fidler PS, Morgenstern DA, Adcock KH, Oohira A, Rogers JH, Fawcett JW (2000) Neurocan is upregulated in injured brain and in cytokine-treated astrocytes. J Neurosci 20: 2427-2438.
Asher RA, Morgenstern DA, Adcock KH, Rogers JH, Fawcett JW (1999) Versican is up-regulated in CNS injury and is a product of O-2A lineage cells. Soc Neurosci Abstr 25: 750.
Bandtlow, CE and Schwab, ME, NI-35/250/nogo-a: a neurite growth inhibitor restricting structural plasticity and regeneration of nerve fibers in the adult vertebrate CNS, Glia, 29 (2000) 175-181.
Bradbury,E.J. et al. NT-3 promotes growth oflesioned adult rat sensory axons ascending in the dorsal columns of the spinal cord. Eur. .I: Neurosci. 11,3873-3883 (1999).
Bradbury,E.J., Bennett,G.S., Moon,L.D.F., Patel,P.N., Fawcett,J.W., and McMahon,S.B. (2000a) Chondroitinase ABC delivered to the site of a spinal cord injury upregulates GAP-43 expression in dorsal root ganglion neurons, Soc. Neurosci. Abstr., 26: 860.
Bradbury,E.J., McMahon,S.B. & Ramer,M.S. (2000b) Keeping in touch: sensory neurone regeneration in the CNS. Trends Pharmacol. Sci. 21, 389-394.
Bradbury, E. J., Moon, L. D. F., Popat, R. J., King, V. R., Bennett, G. S., Patel, P. N., Fawcett, J. W., and McMahon, S. B. (2002) Chondroitinase ABC promotes functional recovery following spinal cord injury. Nature, 416: 636-640.
Bregman,B.S. et al. Recovery from spinal cord injury mediated by antibodies to neurite growth inhibitors. Nature 378,498-501 (1995).
Chong,M.S. et al. GAP-43 expression in primary sensory neurons following central axotomy. J. Neurosci. 14, 4375-4384 (1994).
Davies,S.J., Goucher,D.R., Doller,C. & Silver,J. Robust regeneration of adult sensory axons in degenerating white matter of the adult rat spinal cord..J. Neurosci. 19,5810-5822 (1999).
DeWitt DA, Richey PL, Praprotnik D, Silver J, Perry G (1994) Chondroitin sulfate proteoglycans are a common component of neuronal inclusions and astrocytic reaction in neurodegenerative diseases. Brain Res 656: 205-209.
Fawcett JW, Asher RA (1999) The glial scar and CNS repair. Brain Res Bull 49: 377-391.
Fidler PS, Schuette K, Asher RA, Dobbertin A, Thornton SR, Calle-Patino Y, Muir E, Levine JM, Geller HM, Rogers JH, Faissner A, Fawcett JW (1999) Comparing astocytic cells lines that are inhibitory or permissive for axon growth: the major axon - inhibitory proteoglycan is NG2. J Neurosci 19: 8778-8788.
Hill,C.E., Beattie,M.S. & Bresnahan,J.C. Degeneration and sprouting of identified descending supraspinal axons after contusive spinal cord injury in the rat. Exp. Neurol. 171, 153-169 (2001).
Koppe G, Bruckner G, Brauer K, Hartig W, and Bigl V (1997) Developmental patterns of proteoglycan-containing extracellular matrix in perineuronal nets and neuropil of the postnatal rat brain, Cell Tissue Res., 288 33-41.
Kunkel-Bagden,E., Dai,H.N. & Bregman,B.S. Methods to assess the development and recovery of locomotor function after spinal cord injury in rats. Exp. Neurol. 119, 153-164 (1993).
Lander,C., Kind,P., Maleski,M., and Hockfield,S., A family of activity-dependent neuronal cell-surface chondroitin sulfate proteoglycans in cat visual cortex, J. Neurosci., 17, 1928-1939 (1997).
Levine,J.M. Increased expression of the NG2 chondroitin-sulfate proteoglycan after brain injury. J. Neurosci. 14,4716-4730 (1994).
Lodovichi C, Berardi N, Pizzorusso T, Maffei L (2000) Effects of neurotrophins on cortical plasticity: Same or different? Journal Of Neuroscience 20: 2155-2165.
Maffei L, Berardi N, Domenici L, Parisi V, Pizzoruso T (1992) Nerve growth factor (NGF) prevents the shift in ocular dominance distribution of visual cortical neurons in monocularly deprived rats. J.Neurosci. 12 (12):4651-4662
McKeon,R.J., Schreiber,R.C., Rudge,J.S. & Silver,J. Reduction of neurite outgrowth in a model of glial scarring following CNS injury is correlated with the expression of inhibitory molecules on reactive astrocytes. J. Neurosci. 11, 3398-3411 (1991).
McKeon,R.J., Hoke,A. & Silver,J. Injury-induced proteoglycans inhibit the potential for laminin-mediated axon growth on astrocytic scars. Exp. Neurol. 136, 32-43 (1995).
McKeon,R.J., Jurynec,M.J. & Buck,C.R. The chondroitin sulfate proteoglycans neurocan and phosphacan are expressed by reactive astrocytes in the chronic CNS glial scar. J. Neurosci. 19, 10778-10788 (1999).
Moon LDF, Asher RA, Rhodes KE, Fawcett JW (2001) Regeneration of CNS axons back to their original target following treatment of adult rat brain with chondroitinase ABC. Nat Neurosci 4: 465-466.
Mori,M., Kose,A., Tsujino,T. & Tanaka,C. Immunocytochemical localization of protein kinase C subspecies in the rat spinal cord: light and electron microscopic study. J. Comp Neurol. 299, 167-177 (1990).
Murray,M. Strategies and mechanisms of recovery after spinal cord injury. Adv. Neurol. 72,219-225 (1997).
Niederost,B.P ., Zimmermann,D.R., Schwab,M.E. & Bandtlow,C.E. Bovine CNS myelin contains neurite growth-inhibitory activity associated with chondroitin sulfate proteoglycans. J. Neurosci. 19,8979-8989 (1999).
Pasterkamp,R.J., Anderson,P.N. & Verhaagen,J. Peripheral nerve injury fails to induce growth of lesioned ascending dorsal column axons into spinal cord scar tissue expressing the axon repellent Semaphorin3A. Eur. J. Neurosci. 13, 457-471 (2001).
Ramer,M.S., Priestley,J.V. & McMahon,S.B. Functional regeneration of sensory axons into the adult spinal cord. Nature 403,312-316 (2000).
Rhodes,K.E., Moon,L.D.F., and Fawcett,J.W. (2000) Inhibiting cell proliferation in glial scar formation: effects on axon regeneration in the CNS. Soc. Neurosci. Abstr., 26 854.
Smith-Thomas L, Fok-Seang J, Stevens J, Du J-S, Muir E, Faissner A, Geller HM, Rogers JH, Fawcett JW (1994) An inhibitor of neurite outgrowth produced by astrocytes. J Cell Sci 107: 1687-1695.
Smith-Thomas L, Stevens J, Fok-Seang J, Muir E, Faissner A, Rogers JH, Fawcett JW (1995) Increased axon regeneration in astrocytes grown in the presence of proteoglycan synthesis inhibitors. J Cell Sci 108: 1307-1315.
Thallmair,M. et al. Neurite growth inhibitors restrict plasticity and functional recovery following corticospinal tract lesions. Nat. Neurosci. 1, 124-131 (1998).
Wall,P.D. & Lidierth,M. Five sources of a dorsal root potential: their interactions and origins in the superficial dorsal horn. J Neurophysiol. 78,860-871 (1997).
Yamada,H. et al. The brain chondroitin sulfate proteoglycan brevican associates with astrocytes ensheathing cerebellar glomeruli and inhibits neurite outgrowth from granule neurons. J. Neurosci. 17,7784-7795 (1997).
Zuo,J., Neubauer,D., Dyess,K., Ferguson, T.A. & Muir,D. Degradation of chondroitin sulfate proteoglycan enhances the neurite-promoting potential of spinal cord tissue. Exp. Neurol. 154,654-662 (1998).

The following numbered statements, describing aspects of our proposals, are part of the description.
1. A method of promoting neuronal plasticity in the CNS of a mammal, the method comprising administering to the CNS of the mammal an agent that reduces the inhibitory properties of chondroitin sulphate proteoglycans.
2. The use of an agent which reduces the inhibitory properties of chondroitin sulphate proteoglycan, in the preparation of a medicament for promoting neuronal plasticity in a mammal.
3. The method or use of statement 1 or statement 2, wherein the promotion of neuronal plasticity is promotion of neuronal plasticity in the spinal cord or structure whose component cell bodies are located in, or have primary synapses in, the spinal cord.
4. The method or use of any preceding statement, wherein the promotion of neuronal plasticity is following spinal cord injury.
5. The method or use of statement 4, wherein the spinal cord injury is an injury caused by accident, assault, tumour, surgery, or an intervertebral disc or bone abnormality.
6. The method or use of statement 1 or statement 2, wherein the promotion of neuronal plasticity is promotion of neuronal plasticity in the brain or structure whose component cell bodies are located in, or have primary synapses in, the brain.
7. The method or use of statement 6, wherein the promotion of neuronal plasticity is promotion of neuronal plasticity in the cortex.
8. The method or use of statement 7, wherein the promotion of neuronal plasticity is following stroke, brain injury, multiple sclerosis or a neurodegenerative disease that affects the cortex.
9. The method or use of statement 8, wherein said brain injury is injury caused by assault, accident, tumour, or surgery.
10. The method or use of statement 9, wherein said tumour is a brain tumour or a non-brain tumour that affects the brain.
11. The method or use of statement 8, wherein said neurodegenerative disease that affects the cortex is Alzheimer's.
12. The method or use of any preceding statement, wherein the agent that reduces the inhibitory properties of chondroitin sulphate proteoglycans is an agent that reduces the neurite-outgrowth inhibitory properties of chondroitin sulphate proteoglycans.
13. The method or use of any preceding statement, wherein the agent that reduces the inhibitory properties of chondroitin sulphate proteoglycans is an agent selected from the group consisting of:
   an agent that interacts with one or more chondroitin sulphate proteoglycans (CSPGs) to inhibit their inhibitory properties,
   an agent that eliminates one or more CSPGs,
   an agent that reduces the production of one or more CSPGs,
   an agent that interacts with hyaluronan to inhibit its ability to bind to CSPGs,
   an agent that eliminates hyaluronan,
   an agent that reduces the production of hyaluronan, and
   an agent that blocks, destroys or reduces the synthesis of a receptor by which hyaluronan is anchored to the surface of cells in the CNS.
14. The method or use of any preceding statement, wherein the agent that reduces the inhibitory properties of chondroitin sulphate proteoglycans is an agent selected from the group consisting of:
   an agent that removes, digests, binds to and blocks, or prevents the synthesis of one or more chondroitin sulphate proteoglycans,
   an agent that removes, digests, binds to and blocks, or prevents the synthesis of hyaluronan, and
   an agent that removes, digests, binds to and blocks, or prevents the synthesis of one or more hyaluronan receptors.
15. The method or use of any preceding statement, wherein the agent that reduces the inhibitory properties of chondroitin sulphate proteoglycans is an agent that removes, digests, binds to and blocks, or prevents the synthesis of the glycosaminoglycan chains of one or more chondroitin sulphate proteoglycans.
16. The method or use of any one of statements 13-15, wherein the agent is an antibody.
17. The method or use of statement 16, wherein the antibody is selected from the group consisting of:
   an antibody that is capable of binding one or more CSPGs to inhibit their inhibitory properties,
   an antibody that is capable of binding hyaluronan to block the attachment of CSPGs thereto,
   an antibody that is capable of binding a hyaluronan receptor to block the attachment of hyaluronan thereto.
18. The method or use of any one of statements 13-15, wherein the agent is an agent selected from the group consisting of:
   an enzyme capable of digesting CSPGs, and
   an enzyme capable of digesting hyaluronan.
19. The method or use of statement 18, wherein the enzyme is capable of digesting and/or removing the sulphate groups from the glycosaminoglycan chains of CSPGs.
20. The method or use of statement 19, wherein the enzyme has chondroitinase activity.
21. The method or use of statement 20, wherein the enzyme is chondroitinase.
22. The method or use of statement 21, wherein the enzyme is chondroitinase ABC, chondroitinase B or chondroitinase AC.
23. The method or use of any one of statements 18 to 20, wherein the enzyme is hyaluronidase.
24. The method or use of any one of statements 13-15, wherein the agent is selected from the group consisting of:
   an inhibitor of one or more of the enzymes responsible for CSPG synthesis, and
   an inhibitor of one or more of the enzymes responsible for hyaluronan synthesis.
25. The method or use of statement 24, wherein the agent that reduces the production of one or more CSPGs is an inhibitor of one or more of the enzymes involved in the production of the glycosaminoglycan chains of CSPGs and/or their attachment to the CSPG protein moiety.
26. The method or use of statement 25, wherein the agent that reduces the production of one or more CSPGs is an inhibitor of one or more chondroitin sulfotransferases.
27. The method or use of statement 26, wherein the agent that reduces the production of one or more CSPGs is an inhibitor of chondroitin 6-O-sulfotransferase-1 and/or chondroitin 6-O-sulfotransferase-2 and/or uronyl-2-sulfotransferase.
28. The method or use of statement 25, wherein the inhibitor is a β-D-xyloside.
29. The method or use of any one of statements 13-15, wherein the agent is an agent that kills one or more of the non-neuronal cell types that produces CSPGs, and/or hyaluronan and/or hyaluronan receptors.
30. The method or use of statement 29, wherein the agent is an agent that kills oligodendrocyte precursors.
31. The method or use of statement 30, wherein the agent is cytosine arabinoside.
32. The method or use of any one of statements 13-15, wherein the agent is an antisense or dsRNA oligonucleotide capable of inhibiting the production of a CSPG core protein and/or a hyaluronan receptor.
33. The method or use of any one of statements 1-15, wherein the agent is a sugar molecule that is capable of binding to and blocking, or that is a competitive antagonist of the GAG chain of one or more CSPGs and/or hyaluronan.
34. An agent that reduces the inhibitory properties of chondroitin sulphate proteoglycans, for use in a method of promoting neuronal plasticity in a mammal.
35. The agent of statement 34, wherein the promotion of neuronal plasticity is as defined in any one of statements 3-10.
36. The agent of statement 34 or statement 35, which is as defined in any one of statements 13-33.
37. A method of identifying an agent useful in promoting neuronal plasticity, the method comprising the steps of:
   (a) bringing into contact (i) a candidate agent and (ii) a substance selected from the group consisting of a CSPG, a CSPG glycosaminoglycan chain, a CSPG synthetic enzyme, a CSPG synthetic enzyme substrate, hyaluronan, a hyaluronan synthetic enzyme, and a hyaluronan receptor;
   (b) determining binding between (i) and (ii); and
   (c) following a positive determination of binding, assaying the candidate agent for the ability to promote neuronal plasticity and/or to promote functional recovery following CNS damage; and optionally
   (d) optimising the candidate agent for in vivo use by further steps including generating mimetics of the candidate agent and repeating steps (a) and (b) and/or repeating step (c) with the mimetic as the candidate agent.
38. A method of identifying an agent useful in promoting neuronal plasticity, the method comprising the steps of:
   (a) bringing into contact (i) a candidate agent and (ii) a substance selected from the group consisting of CSPG, a CSPG glycosaminoglycan chain, and hyaluronan;
   (b) determining the ability of (i) to digest (ii); and
   (c) following a positive determination of digestion, assaying the candidate agent for the ability to promote neuronal plasticity and/or to promote functional recovery following CNS damage; and optionally
   (d) optimising the candidate agent for in vivo use by further steps including generating mimetics of the candidate agent and repeating steps (a) and (b) and/or repeating step (c) with the mimetic as the candidate agent.
39. The method of statement 37 or statement 38, further including a step preceding step (c) of assaying the candidate agent for the ability to reduce the neurite-outgrowth and/or axon regeneration inhibitory properties of CSPGs following CNS damage.
40. The use of a CSPG, a CSPG glycosaminglycan chain, a CSPG synthetic enzyme, a CSPG synthetic enzyme substrate, hyaluronan, a hyaluronan synthetic enzyme, or a hyaluronan receptor in the identification of an agent useful in promoting neuronal plasticity.
41. The method or use of any one of statements 37-40, wherein the candidate agent is a mimetic of a β-D-xyloside.
42. A method comprising the step, following identification of an agent useful in promoting neuronal plasticity according to any one of statements 37-41, of formulating the agent with one or more conventional excipients acceptable for pharmaceutical or veterinary use.

## Claims

1. Chondroitinase for use in the treatment of central nervous system (CNS) damage other than spinal cord injury.

2. The use of chondroitinase in the preparation of a medicament for use in the treatment of CNS damage other than spinal cord injury.

3. The chondroitinase or use of claim 1 or claim 2, wherein the CNS damage is stroke, brain injury, multiple sclerosis or a neurodegenerative disease that affects the cortex.

4. The chondroitinase or use of claim 3, wherein said brain injury is injury caused by assault, accident, tumour, or surgery.

5. The chondroitinase or use of claim 4, wherein said tumour is a brain tumour or a non-brain tumour that affects the brain.

6. The chondroitinase or use claim 3, wherein said neurodegenerative disease that affects the cortex is Alzheimer's.

7. The chondroitinase or use of any one of claims 1 to 6, wherein the chondroitinase is chondroitinase ABC, chondroitinase B or chondroitinase AC.

8. A method of identifying an agent useful in treatment of CNS damage other than spinal cord injury, the method comprising the steps of:
(a) bringing into contact (i) a candidate agent and (ii) a substance selected from the group consisting of a chondroitin sulphate proteoglycan (CSPG), a CSPG glycosaminoglycan chain, a CSPG synthetic enzyme, a CSPG synthetic enzyme substrate, hyaluronan, a hyaluronan synthetic enzyme, and a hyaluronan receptor; and,
(b) determining binding between (i) and (ii).

9. A method of identifying an agent useful in treatment of CNS damage other than spinal cord injury, the method comprising the steps of:
(a) bringing into contact (i) a candidate agent and (ii) a substance selected from the group consisting of chondroitin sulphate proteoglycan (CSPG), a CSPG glycosaminoglycan chain, and hyaluronan; and,
(b) determining the ability of (i) to digest (ii).

10. The method of claim 8 or claim 9, further including a step of assaying the candidate agent for the ability to reduce the neurite-outgrowth and/or axon regeneration inhibitory properties of CSPGs.

11. The method of any one of claims 8 to 10 comprising;
(c) following a positive determination of binding or digestion, assaying the candidate agent for the ability to promote functional recovery following CNS damage; and optionally
(d) optimising the candidate agent for in vivo use by further steps including generating mimetics of the candidate agent and repeating steps (a) and (b) and/or repeating step (c) with the mimetic as the candidate agent.

12. A method of identifying an agent useful in treatment of CNS damage other than spinal cord injury, the method comprising the steps of:
(a) providing a candidate agent which binds to or digests a substance selected from the group consisting of chondroitin sulphate proteoglycan (CSPG), a CSPG glycosaminoglycan chain, and hyaluronan,
(b) assaying the candidate agent for the ability to promote functional recovery following CNS damage; and optionally,
(c) optimising the candidate agent for in vivo use by further steps including generating mimetics of the candidate agent and repeating step (b) with the mimetic as the candidate agent.

13. The use of a CSPG, a CSPG glycosaminglycan chain, a CSPG synthetic enzyme, a CSPG synthetic enzyme substrate, hyaluronan, a hyaluronan synthetic enzyme, or a hyaluronan receptor in the identification of an agent useful in treatment of CNS damage other than spinal cord injury.

14. The method or use of any one of claims 8 to 14, wherein the candidate agent is a mimetic of a β-D-xyloside.

15. A method comprising the step, following identification of an agent useful in treatment of CNS damage other than spinal cord injury, according to any one of claims 8 to 14, of formulating the agent with one or more conventional excipients acceptable for pharmaceutical or veterinary use.
